# EUROPEAN PATENT APPLICATION

(11) **EP 3 238 533 A1**
(43) Date of publication of application: **01.11.2017**
(21) Application number: 16382188.7
(22) Date of filing: 28.04.2016
(51) Int. Cl.: A01H 1/04, A01H 5/08

(54) **BEGOMOVIRUS-RESISTANT MELON PLANTS**

(71) Applicant: Semillas Fito, S. A., 08019 Barcelona (ES)
(72) Inventor: KOUMPROGLOU, Rachil, 08019 Barcelona (ES); FERNANDEZ ZURRO, Marta, 08019 Barcelona (ES); FERNANDEZ, Juan Antonio, 08019 Barcelona (ES); JAHRMANN, Torben, 08019 Barcelona (ES)
(74) Representative: Pons

(57) **Abstract**

The invention relates to a begomovirus resistant plant of the species *Cucumis melo,* said plants comprising genetic element, incorporated by introgression, from a plant of the species *Cucumis melo L.* var. *momordica* (NCIMB 42557), which genetic element comprises a begomovirus-resistant-conferring QTL, or a begomovirus-resistant-conferring part thereof linked to at least one marker located on the chromosome equivalent to linkage group (LG) 5 of NCIMB 42557, wherein said plant is not NCIMB 42557. Particularly, the begomovirus is the Tomato leaf curl New Delhi virus (ToLCNDV).

SEQUENCE LISTING

<110>  Semillas Fito, S.A.

<120>  BEGOMOVIRUS-RESISTANT MELON PLANTS

<130>  EP2096.9

<160>  24

<170>  PatentIn version 3.5

<210>  1
<211>  121
<212>  DNA
<213>  Cucumis melo

<220>
<221>  variation
<222>  (61)..(61)
<223>  T

<400>  1
ttgcaaggtg ggctgagatg atgaggaaga gggttaagag aagaaatcct acaaacttta      60

ctaatggtga ccatagccct tagaatctca tttaattaaa caccattttt cacttaatcc      120

a                                                                      121

<210>  2
<211>  101
<212>  DNA
<213>  Cucumis melo

<220>
<221>  variation
<222>  (51)..(51)
<223>  C

<400>  2
gtgcatttca tgaaaaaaat agaaactaaa aagtgtgaat tttagaaagt tcgacttcaa      60

attgaaataa aaaacttaaa atttaaaaaa taaaattgta a                          101

<210>  3
<211>  101
<212>  DNA
<213>  Cucumis melo

<220>
<221>  variation
<222>  (51)..(51)
<223>  T

<400>  3
taaaccaact ataacttagg gttgtataaa ttttcaaatg tctgtttcaa gggttacttg      60

cataaatgac aaaaaaaaaa aaaaacgtaa aataaaagta t                          101

<210>  4
<211>  101
<212>  DNA
<213>  Cucumis melo


<220>
<221>  variation
<222>  (51)..(51)
<223>  T

<400>  4
aaattttctc tctaatacca tcttaatttt tcaattttct ttctgttaca catatgaatt      60

ttacaaggta tcaaatttca cctcaaccaa aaaaatgatt t                          101


<210>  5
<211>  101
<212>  DNA
<213>  Cucumis melo


<220>
<221>  variation
<222>  (51)..(51)
<223>  T

<400>  5
gagagtaatt aacacttagg tataattacg acgaataaac tacaaaagta gtttgaattt      60

gtgttttttt gagacacaaa ggttttttta aaaatttata t                          101


<210>  6
<211>  196
<212>  DNA
<213>  Cucumis melo


<220>
<221>  variation
<222>  (101)..(101)
<223>  G

<400>  6
gcctatcagg agtgaactca atctcaatgg acatgaaaga gaagaagctg acagtaacag      60

gagacgtaga tccagtagtg attgtaagca aattaaggaa aatttgtcat acaacaatag     120

tttccgttgg accagagaaa gaagagaaga aaccagaacc aaagaaagat gaaccaaaga     180

aggaagatcc aaaaaa                                                      196


<210>  7
<211>  49
<212>  DNA
<213>  Artificial Sequence

<220>

<223>  Primer A1 for the detection of SNP Cm_0205

<400>  7
aaggtgacca agttcatgct gttaagagaa gaaatcctac aaactttac                49


<210>  8
<211>  51
<212>  DNA
<213>  Artificial Sequence

<220>
<223>  Primer A2 for the detection of SNP CM_0205

<400>  8
gaaggtcgga gtcaacggat tggttaagag aagaaatcct acaaacttta t             51


<210>  9
<211>  26
<212>  DNA
<213>  Artificial Sequence

<220>
<223>  Primer C1 for the detection of CM_0205

<400>  9
gagattctaa gggctatggt caccat                                         26


<210>  10
<211>  54
<212>  DNA
<213>  Artificial Sequence

<220>
<223>  Primer A1 for the detection of SNP MEL_SNP_2208

<400>  10
gaaggtgacc aagttcatgc taattttaag ttttttattt caatttgaag tcga          54


<210>  11
<211>  54
<212>  DNA
<213>  Artificial Sequence

<220>
<223>  Primer A2 for the detection of SNP MEL_SNP_2208

<400>  11
gaaggtcgga gtcaacggat taattttaag ttttttattt caatttgaag tcgg          54


<210>  12
<211>  30
<212>  DNA
<213>  Artificial Sequence

<220>
<223>  Primer C1 for the detection of SNP MEL_SNP_2208

<400>  12

agaaactaaa aagtgtgaat tttagaaagt                                    30

<210>  13
<211>  53
<212>  DNA
<213>  Artificial Sequence

<220>
<223>  Primer A1 for the detection of SNP MEL_SNP_2224

<400>  13
gaaggtgacc aagttcatgc tttttttttt tttgtcattt atgcaagtaa ccc             53

<210>  14
<211>  52
<212>  DNA
<213>  Artificial Sequence

<220>
<223>  Primer A2 for the detection of SNP MEL_SNP_2224

<400>  14
gaaggtcgga gtcaacggat tttttttttt ttgtcattta tgcaagtaac ca              52

<210>  15
<211>  30
<212>  DNA
<213>  Artificial Sequence

<220>
<223>  Primer C1 for the detection of SNP MEL_SNP_2224

<400>  15
agggttgtat aaattttcaa atgtctgttt                                    30

<210>  16
<211>  54
<212>  DNA
<213>  Artificial Sequence

<220>
<223>  Primer A1 for the detection of SNP MEL_SNP_4105

<400>  16
gaaggtgacc aagttcatgc tcatcttaat ttttcaattt tctttctgtt acac            54

<210>  17
<211>  54
<212>  DNA
<213>  Artificial Sequence

<220>
<223>  Primer A2 for the detection of SNP MEL_SNP_4105

<400>  17
gaaggtcgga gtcaacggat tcatcttaat ttttcaattt tctttctgtt acat            54

<210>  18
<211>  30
<212>  DNA
<213>  Artificial Sequence

<220>
<223>  Primer C1 for the detection of SNP MEL_SNP_4105

<400>  18
tggttgaggt gaaatttgat accttgtaaa                                        30


<210>  19
<211>  53
<212>  DNA
<213>  Artificial Sequence

<220>
<223>  Primer A1 for the detection of SNP MEL_SNP_4114

<400>  19
gaaggtgacc aagttcatgc tgtataatta cgacgaataa actacaaaag tag              53


<210>  20
<211>  53
<212>  DNA
<213>  Artificial Sequence

<220>
<223>  Primer A2 for the detection of SNP MEL_SNP_4114

<400>  20
gaaggtcgga gtcaacggat tgtataatta cgacgaataa actacaaaag tat              53


<210>  21
<211>  30
<212>  DNA
<213>  Artificial Sequence

<220>
<223>  Primer C1 for the detection of SNP MEL_SNP_4114

<400>  21
cctttgtgtc tcaaaaaaac acaaattcaa                                        30


<210>  22
<211>  50
<212>  DNA
<213>  Artificial Sequence

<220>
<223>  Primer A1 for the detection of SNP SFMe066

<400>  22
gaaggtgacc aagttcatgc tcagtagtga ttgtaagcaa attaaggaaa                  50


<210>  23
<211>  50
<212>  DNA

<213>  Artificial Sequence

<220>
<223>  Primer A2 for the detection of SNP SFMe066

<400>  23
gaaggtcgga gtcaacggat tcagtagtga ttgtaagcaa attaaggaag                50


<210>  24
<211>  29
<212>  DNA
<213>  Artificial Sequence

<220>
<223>  Primer C1 for the detection of SNP SFMe066

<400>  24
gtccaacgga aactattgtt gtatgacaa                                       29

## Description

The invention relates to a begomovirus-resistant plant, preferably a tomato leaf curl New Delhi virus (ToLCNDV) resistant-plants, preferably from species *Cucumis melo,* said plant comprising a genetic element, incorporated by introgression from a plant whose seeds where deposited with the NCIMB under accession number NCIMB 42557, wherein the genetic element comprises a begomovirus-resistant-conferring quantitative trait locus (QTL) located on preferably linkage group (LG) 5 and further on LG 9, or a begomovirus-resistant-conferring part thereof.

### BACKGROUND ART

ToLCNDV is a bipartite begomovirus that belongs to *Geminividae* family and is transmitted by the whitefly *Bemisia tabaci* in a persistent mode. To date, it is not known if the virus can be transmitted by contact or by seeds. ToLCNDV was first detected in tomato *(Solanum lycopersicum L.)* in north India in 1995. During the next years the virus was spread in other parts of the country or neighboring countries and infected particularly vegetables of the *Cucurbitaceae* and *Solanaceae* families such as cucumber and melon *(Cucumis sativus L.* and *Cucumis melo L.),* watermelon *(Citrullus annatus* [Thunb.] Matsum. and Nakai), bottlegourd *(Lagenaria* spp), sponge and ridge gourds (*Luffaspp*)*,* bitter gourd *(Momordica charantia L.),* waxgourd *(Benincasa hispida* [Thunb.] Cogn.), pumpkin (*Cucurbita maxima Duchesne),* chilli pepper *(Capsicumspp.)* and potato *(Solanum tuberosum L.).* In In September 2012, symptoms caused by ToLCNDV were first observed on courgette (*Cucurbita pepo* var. *giromontiina*) in Murcia, Spain. In May 2013, similar symptoms were reported in Almeria province, and by autumn 2013, the disease was widespread in both Spanish regions. In January 2015, the virus was detected for the first time in Tunisia, causing a severe disease on melon, cucumber and courgette cultivated under plastic tunnels.

The main symptoms that causes are stunting plants, yellow leaf mosaic, curling leaves and vein swelling. On cucurbit fruit, rough skin and longitudinal cracking can be observed. If the virus infection occurs at an early stage, affected plants are severely stunted and fruit production is largely deteriorated, if not vanished at all. Control measures against ToLCNDV are very limited and mainly rely on whitefly control, cultivation under insect-proof greenhouses, elimination of infected plants, and avoidance of the most susceptible cultivars, all of them with limited success. The use of genetically resistant cultivars is a good option for ToLCNDV control and would allow grows of cultivars in open field conditions. A first report on this ground involved a monogenic resistance that has been reported in a breeding line of sponge gourd *(Luffacylindrica M. Roem.).*

Genetic material from accessions that comprises the genetic information responsible for the ToLCNDV resistance could be introgressed into commercial cultivars, but this procedure is not always successful. In this sense, it is not known which specific accession provides the best source of resistance. If the result is a cultivar exhibiting partial resistance, the result of the market introduction of such a cultivar may lead to the development of resistance-breaking viral strains. Furthermore, the possibility of stably fixing the resistance trait in the genome of the target plant remains to be determined. Thus, choosing a particular source of resistance does not guarantee success and may even hold various risks. In addition, the introgression itself involves a substantial breeding effort, and includes the development and performance of bioassays to follow resistant offspring plants. In effect, the development of a resistant cultivar is commercially costly undertaking and any program may be early abandoned when results fail to precipitate.

In order to reduce the uncertainties and work involved in developing a resistant cultivar, it would be beneficial to have a simple genomic marker for the resistance trait. Such a marker could then be used in marker assisted selection (MAS) procedures as part of a dedicated breeding program. Whether such a marker can be found is partly determined by genomic structure of the resistance trait. If the trait is multi-genic, it is not likely that a single marker is found that may reliably be used in MAS procedures. Moreover, the development of the marker(s) themselves then mounts to a significant undertaking, possibly overshadowing the costs and time involved in a straightforward breeding program. However, once a suitable resistance source is identified and a marker is developed, the new resistant plants can be easily traced, which increases their commercial value.

### SUMMARY OF THE INVENTION

The present invention now relates in a first aspect to a begomovirus resistant plant of the family *Cucurbitaceae,* preferably from the genera *Cucumis,* and more preferably from the species *Cucumis melo,* wherein said plant comprising an introgression of a genetic element derived from a plant of the species *Cucumis melo* L. var. *momordica* grown from seed that was deposited with the NCIMB under accession number NCIMB 42557, which genetic element comprises a begomovirus-resistance-conferring QTL or a begomovirus-resistance-conferring part thereof, located on linkage group 5, preferably between physical positions 24,008,941 bp and 24,895,385 bp, on the basis of the melon genome version CM3.5, as further defined below, with the condition that the begomovirus resistant plant of the present invention is not *Cucumis melo* L. var. *momordica* NCIMB 42557.

A representative sample of seed of *Cucumis melo* L. var. *momordica* comprising the QTLs for begomovirus-resistance, preferably for ToLCNDV-resistance were deposited by Semillas Fitó S.A.U. on 16 March 2016 at the NCIMB Ltd., Ferguson Building, Craibstone Estate, Bucksburn, Aberdeen AB21 9YA, United Kingdom (NCIMB), according to the Budapest Treaty, under the Expert Solution (EPC2000, Rule 32(1)). Seed were given the following deposit number: NCIMB 42557.

The present inventors have now found that the resistance in *Cucumis melo* var. *momordica* is linked to a defined genetic region or QTL. A melon plant of the invention comprises a genetic element derived from a plant of the species *Cucumis melo* var. *momordica,* in particular from NCIMB 42557, which genetic element comprises the begomovirus-resistance-conferring QTLs, preferably the ToLCNDV-resistance-conferring QTLs as identified by the present invention.

A plant of the present invention is preferably a *Cucumis melo* cultivar, but is not necessarily restricted to a specific strain. A plant of the present invention is preferably a *Cucumis melo* plant having commercially valuable characteristics, including, but not limited to melon plants having commercially valuable fruit or seed characteristics. A begomovirus, preferably a ToLCNDV-resistant plant of the species *Cucumis melo* may be any *Cucumis melo* species, with the proviso that the plant is not NCIMB 42557. In a more preferred embodiment, the ToLCNDV-resistant plant of the present invention is an inbred plant or a hybrid plant. Another preferred embodiment, a plant of the present invention is melon cultivar. In another aspect, the present invention relates to a part of a plant of the invention, such as a seed or a fruit.

The ToLCNDV-resistant *C. melo* plants of the present invention are plants selected from the group consisting of *Cucumis melo var. cantalupensis, Cucumis melo var. reticulates, Cucumis melo var. inodorus, Cucumis melo var. acidulus; Cucumis melo var. aegyptiacus; Cucumis melo var. ameri; Cucumis melo var. duripulposus; Cucumis melo var. hibernus; Cucumis melo var. makuwa* and *Cucumis melo var. microspermus,* more preferably a line thereof that possess commercially desirable characteristics

Said plants of the invention comprise the referred QTLs as defined herein in a form wherein the resistance genes are present on both alleles, preferably in a dominant form. Thus, the plants of the present invention are homozygous for the resistance trait. It should be noted in this respect that although heterozygous plants do not express a recessive trait, new plants that comprise the QTL of the invention, or begomovirus-resistance conferring genes comprised therein, preferably ToLCNDV-resistance conferring genes, in heterozygous form constitute an important intermediate product in a program to develop a resistant cultivar.

The begomovirus, preferably the ToLCNDV-resistance-conferring QTLs of the present invention are preferably associated with markers that are located on the chromosome equivalent of linkage group (LG) 5 (QTL-1) and/or on the chromosome equivalent of linkage group (LG) 9 (QTL-2), more preferably, on the chromosome equivalent of linkage group 5 and linking group 9. As a physical entity, QTLs, preferably the QTL-1 and the QTL-2 are part of a nucleic acid, either isolated or in a genomic background, and are capable of conferring begomovirus-resistance, preferably ToLCNDV-resistance to a plant in the genome of which it are introduced, preferably, in a location of the genome that is homologous to the location in the genome of NCIMB 42557 where they were first detected and as specified herein.

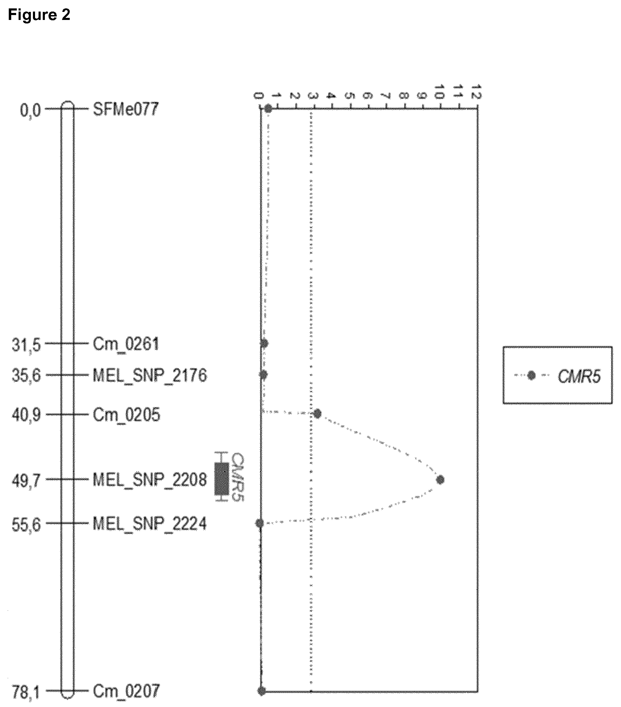

The ToLCNDV-resistance-conferring QTL-1 is located on the chromosome equivalent of linkage group (LG) 5 between physical positions 24,008,941 bp and 24,895,385 bp on the basis of the melon genome version CM3.5, and stretches from position 46.9 to 52.7 cM, preferably at about position 49.7 cM on the Map as presented in Fig. 2. In a preferred embodiment, the ToLCNDV-resistance-conferring QTL-1 is detectable by a molecular marker assay which detects at least the Single Nucleotide Polymorphism (SNP) marker MEL_SNP_2208 comprising the SEQ ID NO: 2. In a more preferred embodiment, the ToLCNDV-resistance-conferring QTL-1 is further detectable by the SNP markers CM_0205 comprising the SEQ ID NO: 1, MEL_SNP_2224 comprising the SEQ ID NO: 3, or any combinations thereof.

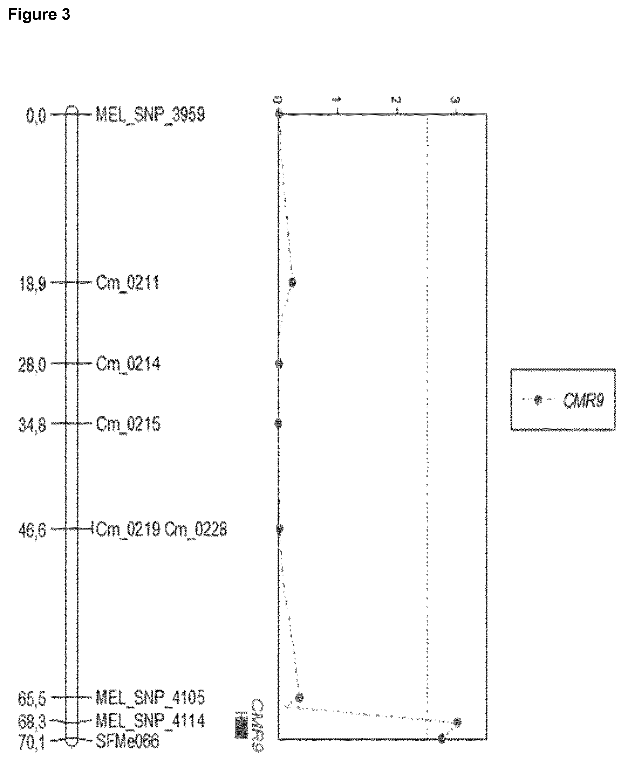

Additionally, the ToLCNDV-resistance-conferring QTL-2 of the present invention is located on the chromosome equivalent of linkage group (LG) 9 between physical positions 22,847,688 bp and 23,556,554 bp, on the basis of the melon genome version CM3.5, or a ToLCNDV-resistant conferring part thereof and stretches from position 66.1 cM to 70.1 cM, preferably at about position 68.3 cM on the Map as presented in Fig. 3. In a preferred embodiment, the ToLCNDV-resistance-conferring QTL-2 is detectable by a molecular marker assay which detects at least the SNP marker MEL_SNP_4114 comprising the SEQ ID NO: 5. In a more preferred embodiment, the ToLCNDV-resistance-conferring QTL-2 is further detectable by the SNP markers MEL_SNP_4105 comprising the SEQ ID NO: 4, SFMe066 comprising the SEQ ID NO: 6, or any combinations thereof.

The markers themselves may be used in aspects of the invention relating to MAS and methods wherein plants having the QTLs are traced. The markers used in such aspects may either be trans, or cis markers. A trans marker indicates a polymorphism resulting from introgression of exogenous (donor) DNA into a recipient plant's genome, which polymorphism is linked in cis with the recipient genome, i.e. linked with the opposite allele. Thus, cis markers are linked with the allele of interest (i.e. from the donor), while trans markers are linked with the opposite allele (i.e. from the recipient). However, both are predictive for the resistant allele encoded by the QTLs of interest.

It is another object of the present invention to provide QTLs that contribute to ToLCNDV-resistance in melon plants *(Cucumis melo).* In a preferred embodiment, the QTLs that contribute to the ToLCNDV-resistance in melon plants are preferably the QTL-1 and the QTL-2 as described above.

It is a further object of the present invention to provide markers that can identify the genetic determinant leading to ToLCNDV-resistance.

In another aspect, the invention relates to a method for producing a plant of the present invention comprising introducing into a begomovirus-susceptible melon plant a genetic element derived from a plant of the species *Cucumis melo* L. var. *momordica,* more preferably from NCIMB 42557, which genetic element comprises the begomovirus-resistance-conferring QTLs, preferably comprising the ToLCNDV-resistance-conferring QTL-1 and/or QTL-2, as described in more detail above. The introduction of the QTLs, or an isolated nucleic acid comprising the QTLs of the invention, or a resistance-conferring part thereof, into a melon plant may be performed by any method known to the artisan. Preferably the introduction is performed by introgression (crossing). In such an embodiment, a method of the present invention comprises the step of crossing a plant NCIMB 42557, or a begomovirus-resistant derivative thereof, preferably a ToLCNDV-resistance-derivative thereof, with a begomovirus-susceptible melon plant; preferably with a ToLCNDV-resistance-derivative, harvesting seed from the cross, growing said seed to produce F1 progeny plants; selfing or backcrossing said F1 progeny plants with the donor or recipient parent to produce F2 seeds; growing said F2 seeds to produce F2 progeny plants; determining the presence in the genome of at least one of said F2 progeny plants of a ToLCNDV-resistance-conferring QTLs, wherein said QTLs are characterized as described in more detail above, wherein the presence of said QTLs are confirmed by detecting the presence in the DNA of said at least one F2 progeny plants of a marker linked to said QTLs.

In another aspect, the present invention further provides a method for identifying a begomovirus, preferably a ToLCNDV resistant *C. melo* plant comprising an introgression fragment located on linkage group (LG) 5, comprising:
a) providing a population of cultivated *C. melo* plants (such as an F2, F3, BC1, BC2, BC1S1 population),
b) screening said population using a molecular marker assay which detects at least one SNP marker selected from the group consisting of: SNP marker MEL_SNP_2208 comprising the SEQ ID NO: 2; CM_0205 comprising the SEQ ID NO: 1, MEL_SNP_2224 comprising the SEQ ID NO: 3, or any combinations thereof; and
c) identifying and/or selecting a plant comprising at least one of the SNP markers selected from the group consisting of: SNP marker MEL_SNP_2208 comprising the SEQ ID NO: 2; CM_0205 comprising the SEQ ID NO: 1, MEL_SNP_2224 comprising the SEQ ID NO: 3, or any combinations thereof.

In a preferred embodiment of the method for identifying a ToLCNDV resistant C. *melo* of the present invention it is characterized by the SNP marker MEL_SNP_2208 comprising the SEQ ID NO: 2 is detected, preferably, by the primers of SEQ ID NO: 10, 11 and 12; the SNP marker CM_0205 comprising the SEQ ID NO: 1 is detected, preferably, by the primers of SEQ ID NO: 7, 8 and 9 and the SNP marker MEL_SNP_2224 comprising the SEQ ID NO: 3 is detected, preferably, by the primers of SEQ ID NO: 13, 14 and 15.

Additionally, the method for identifying a ToLCNDV resistant *C. melo* of the present invention also comprises the identification of the introgression fragment located on linkage group (LG) 9 which is detected by screening the population using a molecular marker assay which detect at least one SNP marker selected from the group consisting of: MEL_SNP_4114 comprising the SEQ ID NO: 5, MEL_SNP_4105 comprising the SEQ ID NO: 4, SFMe066 comprising the SEQ ID NO: 6, or any combinations thereof. In a more preferred embodiment, the SNP marker MEL_SNP_4114 comprising the SEQ ID NO: 5 is detected, preferably, by the primers of SEQ ID NO: 19, 20 and 21; the SNP marker MEL_SNP_4105 comprising the SEQ ID NO: 4 is detected, preferably, by the primers of SEQ ID NO: 16, 17 and 18; and the SNP marker SFMe066 comprising the SEQ ID NO: 6 is detected, preferably, by the primers of SEQ ID NO: 22, 23 and 24.

In another aspect of the present invention, it is further provides a seed, a fruit, a plant cell or a plant part of the transgenic plants as described herein. For example, the present invention provides pollen of the plant, an ovule of the plant, a genetically related plant population comprising the plant, a tissue culture of regenerable cells of the plant. In some embodiments, the regenerable cells are derived from embryos, protoplasts, meristematic cells, callus, pollen, leaves, anthers, stems, petioles, roots, root tips, fruits, seeds, flowers, cotyledons, and/or hypocotyls.

### DESCRIPTION OF THE DRAWINGS

**Figure. 1****.** Represents the frequency distribution of the Disease Index of the forth scoring across the Bc1S1 families.
**Figure. 2****.** Represents a linkage map indicating the relative position of the various markers as described herein. Relative positions on linkage group 5 (LG-5) are provided in cM. Only markers with known positions are indicated.
**Figure. 3****.** Represents a linkage map indicating the relative position of the various markers as described herein. Relative positions on linkage group 9 (LG-9) are provided in cM. Only markers with known positions are indicated.

### DETAILED DESCRIPTION OF THE INVENTION

A first aspect to the present invention relates to a begomovirus resistant plant of the family *Cucurbitaceae,* preferably from the genera *Cucumis,* and more preferably from the species *Cucumis melo,* wherein said plant comprising an introgression of a genetic element derived from a plant of the species *Cucumis melo* L. var. *momordica* deposited at the NCIMB on 16 March 2016 under accession number NCIMB 42557, and which genetic element comprises at least a begomovirus-resistance-conferring QTL or a begomovirus-resistance-conferring part thereof, preferably a ToLCNDV-resistance-conferring QTL, preferably a ToLCNDV-resistance-conferring QTL-1, or a ToLCNDV-resistance-conferring part thereof, located on linkage group (LG) 5 between physical positions 24,008,941 bp and 24,895,385 bp, on the basis of the melon genome version CM3.5, wherein said plant is not NCIMB 42557.

As used herein, the term "plant" refers to any living organism belonging to the kingdom Plantae (i.e., any genus/species in the Plant Kingdom). This includes familiar organisms such as but not limited to trees, herbs, bushes, grasses, vines, ferns, mosses and green algae.

The preferred plants used in the present invention refer to plants of the species *Cucumis melo.*

The term "melon" as used herein refers to the species *Cucumis melo L.* (syn. *Cucumis chito; Cucumis dudaim aegyptiacus; Cucumis flexuosus; Cucumis melo var. acidulus; Cucumis melo var. aegyptiacus; Cucumis melo var. ameri; Cucumis melo var. duripulposus; Cucumis melo var. hibernus; Cucumis melo var. makuwa; Cucumis melo var. microspermus; Cucumis microspermus; Cucumis momordica*) and includes both wild accessions as well as cultivars. *Cucumis melo* is generally considered to consist of the subspecies *Cucumis melo subsp. agrestis* and *Cucumis melo subsp. melo.* The latter is then further sub-divided in the botanical varieties var. *Cantalupensis* (syn. Cantaloupe, muskmelon, netted melon, Persian melon, nutmeg melon), var. *chito,* var. *flexuosus,* var. *indorus,* var. *momordica* (snap melon) and var. *texanus.* The NCIMB 42557 as referred to herein corresponds to *Cucumis melo* var. *momordica,* which has the biological status "wild" and its fruits are edible.

In a preferred embodiment of the present invention, the plant of the species *Cucumis melo* is a melon cultivar, wild accession and/or a plant of the group consisting of: *Cucumis melo var. cantalupensis, Cucumis melo var. reticulates, Cucumis melo var. inodorus, Cucumis melo var. acidulus; Cucumis melo var. conomon; Cucumis melo var. chinensis; Cucumis melo var. aegyptiacus; Cucumis melo var. ameri; Cucumis melo var. duripulposus; Cucumis melo var. hibernus; Cucumis melo var. makuwa and Cucumis melo var. Microspermus.* In a more preferred embodiment, the *C. melo* are selected from the group consisting of: *Cucumis melo var. cantalupensis, Cucumis melo var. reticulates and Cucumis melo var. inodorus.*

The term "NCIMB 42557" is used to indicate the source of the ToLCNDV-resistance QTLs identified herein, and includes the seeds as available from any of the public collections or depository institutions well known to the skilled artisan, as well as ToLCNDV-resistant derivatives thereof. Preferably, the seeds from NCIMB 42557 were deposited by Semillas Fitó S.A.U. on 16 March 2016 at the NCIMB Ltd., Ferguson Building, Craibstone Estate, Bucksburn, Aberdeen AB21 9YA, United Kingdom (NCIMB), according to the Budapest Treaty, under accession number NCIMB 42557.

The term "begomovirus" as used herein refers to a genus of viruses, in the family *Geminiviridae.* They are plant viruses that as a group have a very wide host range, infecting dicotyledonous plants. The virus is obligatory transmitted by an insect vector, which can be the whitefly *Bemisia tabaci* or can be other whiteflies. This vector allows rapid and efficient propagation of the virus because it is an indiscriminate feeder. Virus of the genus of begomovirus including, but not limited to tomato yellow leaf curl China virus (TYLCCNV), tomato yellow leaf curl Gezira virus (TYLCGV), tomato yellow leaf curl Malaga virus (TYLCMaIV), tomato yellow leaf curl Sardinia virus (TYLCSV), tomato yellow leaf curl Thailand virus (TYLCTHV), tomato yellow leaf curl virus (TYLCV), African cassava mosaic virus (ACMV), bean golden mosaic geminivirus (BGMV), cabbage leaf curl virus (CaLCuV), tomato chlorotic mottle virus (ToCMoV), tomato golden mosaic virus (TGMV), tomato mosaic Havana virus (ToMHV), tomato mottle taino virus (ToMoTV), tomato mottle virus (ToMoV), tomato rugose mosaic virus (ToRMV), tomato severe leaf curl virus (ToSLCV), tomato severe rugose virus (ToSRV), cotton leaf crumple or curl viruses (cotton leaf crumple virus (CLCrV), cotton leaf curl Allahabad virus (CLCuAV), cotton leaf curl Gezira virus (ClCuGV), cotton Leaf Curl Kokhran Virus (CLCuKV), cotton leaf curl Multan virus (CLCuMV), cotton leaf curl Rajasthan virus (CLCuRV)), East African cassava mosaic viruses (East African cassava mosaic cassava Cameroon virus (EACMCV), East African cassava mosaic Malawi virus (EACMMV), East African cassava mosaic virus (EACMV), East African cassava mosaic Zanzibar virus (EACMZV)), potato yellow mosaic viruses (potato Yellow Mosaic Panama Virus (PYMPV), potato Yellow Mosaic Trinidad Virus (PYMTV), potato yellow mosaic virus (PYMV)), squash leaf curl viruses (squash leaf curl China virus (SLCCNV), squash leaf curl virus (SLCV), Squash leaf curl Yunnan virus (SLCYV)), sweet potato leaf curl viruses (sweet potato leaf curl Georgia virus (SPLCGV), Sweet potato leaf curl virus (SPLCV)), tobacco leaf curl viruses (Tobacco leaf curl Japan virus (TbLCJV), tobacco leaf curl Yunnan virus (TbLCYNV), Tobacco leaf curl Zimbabwe virus (TbLCZV)), tomato leaf curl viruses (tomato leaf curl Bangalore virus (ToLCBV), tomato leaf curl Bangladesh virus (ToLCBDV), tomato leaf curl Gujarat virus (ToLCGV), tomato leaf curl Karnataka virus (ToLCKV), tomato leaf curl Laos virus (ToLCLV), tomato leaf curl Malaysia virus (ToLCMV), tomato leaf curl New Delhi virus (ToLCNDV), tomato leaf curl Sri Lanka virus (ToLCSLV), tomato leaf curl Taiwan virus (ToLCTWV), tomato leaf curl Vietnam virus (ToLCVV), tomato leaf curl virus (ToLCV)), and the like, but examples are not limited to these examples.

In a preferred embodiment of the present invention, the begomovirus is the Tomato Leaf Curl New Delhi Virus (ToLCNDV).

A "locus" is defined herein as the position that a given gene occupies on a chromosome of a given species.

As used herein, the term "heterozygote" refers to a diploid or polyploid individual cell or plant having different alleles (forms of a given gene) present at least at one locus. As used herein, the term "heterozygous" means a genetic condition existing when different alleles (forms of a given gene) reside at corresponding loci on homologous chromosomes.

As used herein, the term "homozygote" refers to an individual cell or plant having the same alleles at one or more or all loci. When the term is used with reference to a specific locus or gene, it means at least that locus or gene has the same alleles. As used herein, the term "homozygous" means a genetic condition existing when identical alleles reside at corresponding loci on homologous chromosomes. When the terms are used with reference to a specific locus or gene, it means at least that locus or gene has the same alleles.

The term "cultivar" is used herein to denote a plant having a biological status other than a "wild" status, which "wild" status indicates the original non-cultivated, or natural state of a plant or accession. The term "cultivar" (for cultivated variety) includes, but is not limited to, semi-natural, semi-wild, weedy, traditional cultivar, landrace, breeding material, research material, breeder's line, synthetic population, hybrid, founder stock/base population, inbred line (parent of hybrid cultivar), segregating population, mutant/genetic stock, and advanced/improved cultivar. As used herein, the term "cultivated variety" or "cultivar" means a group of similar plants that by structural or genetic features and/or performance can be distinguished from other cultivated varieties within the same species. The term "variety" without any specific indication may refer to both a botanical and a cultivated variety, and to either one depending on the context. Examples of cultivars include such cultivated varieties that belong to the botanical groups *Cucumis melo var. cantalupensis* (the Charantais and Italian cantaloupe group), *Cucumis melo var. reticulatis* (the Galia and Ananas group), and *Cucumis melo var. inodorus* (including Piel de Sapo, Yellow Canary, Branco and Honeydew types). Therefore, a plant of the present invention is preferably a plant of the melon botanical varieties *cantalupensis, reticulatis or inodorus.* The term "var." indicates a *varietas* (a taxonomic level below that of the species). A plant of the present invention is preferably not a *Cucumis melo var. Momordica* plant.

As used herein, the term "hybrid" means any offspring of a cross between two genetically unlike individuals, including but not limited to the cross between two inbred lines that differ in one or more genes.

As used herein, the term "inbred" or "inbred line" refers to a substantially homozygous individual or line.

As used herein, the term "line" is used broadly to include, but is not limited to, a group of plants vegetatively propagated from a single parent plant, via tissue culture techniques or a group of inbred plants which are genetically very similar due to descent from a common parent(s). A plant is said to "belong" to a particular line if it (a) is a primary transformant plant regenerated from material of that line; (b) has a pedigree comprised of a primary transformant plant of that line; or (c) is genetically very similar due to common ancestry (e.g., via inbreeding). In this context, the term "pedigree" denotes the lineage of a plant, e.g. in terms of the sexual crosses affected such that a gene or a combination of genes, in heterozygous (hemizygous) or homozygous condition, imparts a desired trait to the plant.

The "genetic trait' is the trait or characteristic that is conferred by the genetic determinant. The genetic trait can be identified phenotypically, for example by performing a bio-assay. However, also plant stages for which no phenotypic assay can be performed do carry the genetic information that leads to the genetic trait. "Trait' or "phenotypic trait" can be used instead of "genetic trait".

As used herein, the term "population" means a genetically heterogeneous collection of plants sharing a common genetic derivation.

As used herein, the term "offspring" refers to any plant resulting as progeny from a vegetative or sexual reproduction from one or more parent plants or descendants thereof. For instance an offspring plant may be obtained by cloning or selfing of a parent plant or by crossing two parent plants as well as the F1 or F2 or still further generations. An F1 is a first-generation offspring produced from parents at least one of which is used for the first time as donor of a trait, while offspring of second generation (F2) or subsequent generations (F3, F4, etc.) are specimens produced from selfings of F1's, F2's etc. An F1 may thus be (and usually is) a hybrid resulting from a cross between two true breeding parents (true-breeding is homozygous for a trait), while an F2 may be (and usually is) an offspring resulting from self-pollination of said F1 hybrids.

As used herein, the term "plant part" refers to any part of a plant, preferably wherein the plant is a melon plant, including but not limited to single cells and cell tissues such as plant cells that are intact in plants, cell clumps and tissue cultures from which melon plants can be regenerated. Examples of plant parts include, but are not limited to, single cells and tissues from pollen, ovules, leaves, embryos, roots, root tips, anthers, flowers, fruits, stems shoots, and seeds; as well as pollen, ovules, leaves, embryos, roots, root tips, anthers, flowers, fruits, stems, shoots, scions, rootstocks, seeds, protoplasts, calli, and the like.

As used herein, the terms "introgression", "introgressed" and "introgressing" refer to both a natural and artificial process whereby genes of one species, variety or cultivar are moved into the genome of another species, variety or cultivar, by crossing those species. The process may optionally be completed by backcrossing to the recurrent parent. For example, introgression of a desired allele at a specified locus can be transmitted to at least one progeny via a sexual cross between two parents of the same species, where at least one of the parents has the desired allele in its genome. Alternatively, for example, transmission of an allele can occur by recombination between two donor genomes, e.g., in a fused protoplast, where at least one of the donor protoplasts has the desired allele in its genome. The desired allele can be, e.g., detected by a marker that is associated with a phenotype, at a QTL, a transgene, or the like. In any case, offspring comprising the desired allele can be repeatedly backcrossed to a line having a desired genetic background and selected for the desired allele, to result in the allele becoming fixed in a selected genetic background. The process of "introgressing" is often referred to as "backcrossing" when the process is repeated two or more times.

As used herein the terms "genetic engineering", "transformation" and "genetic modification" are all used herein as synonyms for the transfer of isolated and cloned genes into the DNA, usually the chromosomal DNA or genome, of another organism.

The terms "resistant" and "resistance" encompass both partial and full resistance to infection. A begomovirus, preferably a ToLCNDV-susceptible melon plant may either be non-resistant or have low levels of resistance to infection by ToLCNDV.

The term "QTL" is used herein in its art-recognised meaning. The term "QTL associated with resistance to begomovirus, preferably resistance to a ToLCNDV in melon", "QTL associated with resistance to ToLCNDV in melon" as well as the shorter terms "QTL for begomovirus-resistance" or "QTL for ToLCNDV-resistance" refer to a region located on a particular chromosome of melon that is associated with at least one gene that encodes for ToLCNDV-resistance or at least a regulatory region, i.e. a region of a chromosome that controls the expression of one or more genes involved in ToLCNDV-resistance. The phenotypic expression of that gene may for instance be observed as a reduced rate of viral replication and/or as a reduced rate of viral movement through the plant. A QTL may for instance comprise one or more genes of which the products confer the genetic resistance. Alternatively, a QTL may for instance comprise regulatory genes or sequences of which the products influence the expression of genes on other loci in the genome of the plant thereby conferring the begomovirus-resistance, preferably the ToLCNDV-resistance. The QTLs of the present invention, preferably the QTL-1 and the QTL-2 may be defined by indicating its genetic location in the genome of the respective wild *Cucumis* accession using one or more molecular genomic markers. One or more markers, in turn, indicate a specific locus. Distances between loci are usually measured by frequency of crossing-over between loci on the same chromosome and expressed as centimorgan (cM). The further apart two loci are, the more likely that a crossover will occur between them. Conversely, if two loci are close together, a crossover is less likely to occur between them. As a rule, one cM (Kosambi map function (cM)) is approximately equal to 1% recombination between loci (markers) (Lui, 1997). When a QTL can be indicated by multiple markers the genetic distance between the end-point markers is indicative of the size of the QTL.

The term "begomovirus-susceptible recipient melon plant" or "ToLCNDV-susceptible recipient melon plant" is used herein to indicate a melon plant that is to receive DNA obtained from a donor melon plant that comprises a QTL for ToLCNDV-resistance. Said "begomovirus-susceptible recipient melon plant" or "ToLCNDV-susceptible recipient melon plant", may or may not already comprise one or more QTLs for ToLCNDV-resistance, in which case the term indicates a plant that is to receive an additional QTL.

The term "natural genetic background" is used herein to indicate the original genetic background of a QTL. Such a background is the genome of the NCIMB 42557. Thus, NCIMB 42557 represents the natural genetic background of the QTLs of the invention. Conversely, a method that involves the transfer of DNA comprising the QTL(s), or a resistance-conferring part thereof, from chromosome 5 and/or 9 of NCIMB 42557, preferably to the same position on the corresponding chromosome of another melon species, will result in that QTL(s), or said resistance-conferring part thereof, not being in its natural genetic background.

As used herein, the term "linkage group" refers to all of the genes or genetic traits that are located on the same chromosome. Within the linkage group, those loci that are close enough together will exhibit linkage in genetic crosses. Since the probability of crossover increases with the physical distance between genes on a chromosome, genes whose locations are far removed from each other within a linkage group may not exhibit any detectable linkage in direct genetic tests. The term "linkage group" is mostly used to refer to genetic loci that exhibit linked behaviour in genetic systems where chromosomal assignments have not yet been made. Thus, in the present context, the term "linkage group" is synonymous to (the physical entity of) chromosome.

In a preferred embodiment, the begomovirus resistant plant of the present invention, preferably the ToLCNDV resistant plant, it is characterized by the QTL(s) mentioned herein or a part thereof, which are present preferably in homozygous form.

As used herein, the term "molecular marker" refers to an indicator that is used in methods for visualizing differences in characteristics of nucleic acid sequences. Examples of such indicators are restriction fragment length polymorphism (RFLP) markers, amplified fragment length polymorphism (AFLP) markers, single nucleotide polymorphisms (SNPs), insertion mutations, microsatellite markers, sequence-characterized amplified regions (SCARs), cleaved amplified polymorphic sequence (CAPS) markers or isozyme markers or combinations of the markers described herein which defines a specific genetic and chromosomal location. In a preferred embodiment the molecular markers of the invention are SNPs.

The term "melon-specific DNA sequence" indicates a polynucleotide sequence having a nucleotide sequence homology of more than 80%, preferably more than 85%, more preferably more than 90%, even more preferably more than 95%, still more preferably more than 97%, most preferably more than 99% with a sequence of the genome of the species *Cucumis melo* that shows the greatest similarity to it, preferably in the case of markers for the QTLs of the present invention, the part of the DNA sequence of NCIMB 42557 flanking the QTL markers.

The term "nucleotide sequence homology" as used herein denotes the presence of homology between two polynucleotides. Polynucleotides have "homologous" sequences if the sequence of nucleotides in the two sequences is the same when aligned for maximum correspondence. Sequence comparison between two or more polynucleotides is generally performed by comparing portions of the two sequences over a comparison window to identify and compare local regions of sequence similarity. The comparison window is generally from about 20 to 200 contiguous nucleotides. The "percentage of sequence homology" for polynucleotides, such as 50, 60, 70, 80, 90, 95, 96, 97, 98, 99 or 100 percent sequence homology may be determined by comparing two optimally aligned sequences over a comparison window, wherein the portion of the polynucleotide sequence in the comparison window may include additions or deletions (i.e. gaps) as compared to the reference sequence (which does not comprise additions or deletions) for optimal alignment of the two sequences. The percentage is calculated by: (a) determining the number of positions at which the identical nucleic acid base occurs in both sequences to yield the number of matched positions; (b) dividing the number of matched positions by the total number of positions in the window of comparison; and (c) multiplying the result by 100 to yield the percentage of sequence homology. Optimal alignment of sequences for comparison may be conducted by computerized implementations of known algorithms, or by visual inspection. Readily available sequence comparison and multiple sequence alignment algorithms are, respectively, the Basic Local Alignment Search Tool (BLAST) (Altschul et al., 1990; Altschul et al., 1997) and ClustalW programs, both available on the internet. Other suitable programs include, but are not limited to, GAP, BestFit, PlotSimilarity, and FASTA in the Wisconsin Genetics Software Package (Genetics Computer Group (GCG), Madison, WI, USA) (Devereux et al., 1984).

The present invention thus provided a ToLCNDV resistant plant of the species C. *melo* comprising a genetic element, incorporated by introgression from a plant whose seeds where deposited with the NCIMB under accession number NCIMB 42557, wherein the genetic element confers resistance to ToLCNDV, wherein the resistance is characterised by the presence of at least:
- QTL1 or a ToLCNDV-resistance-conferring part thereof, located on Linkage Group (LG) 5 between the physical positions 24,008,941 bp and 24,895,385 bp, on the basis of the melon genome version CM3.5, or a ToLCNDV-resistant conferring part thereof and/or,
- QTL2 or a ToLCNDV-resistance-conferring part thereof, located on Linkage Group (LG) 9 between the physical positions 22,847,688 bp and 23,556,554 bp, on the basis of the melon genome version CM3.5, or a ToLCNDV-resistant conferring part thereof

In a preferred embodiment, the ToLCNDV resistant plant of the present invention is characterised by the presence of at least the QTL1, located on Linkage Group (LG) 5 between the physical positions 24,008,941 bp and 24,895,385 bp, on the basis of the melon genome version CM3.5, or a ToLCNDV-resistant conferring part thereof.

In a more preferred embodiment, the ToLCNDV resistant plant of the present invention is characterised by the presence of the QTL1, located on Linkage Group (LG) 5 between the physical positions 24,008,941 bp and 24,895,385 bp, on the basis of the melon genome version CM3.5, or a ToLCNDV-resistant conferring part thereof, and the QTL2, located on Linkage Group (LG) 9 between the physical positions 22,847,688 bp and 23,556,554 bp, on the basis of the melon genome version CM3.5, or a ToLCNDV-resistant conferring part thereof.

As it is mentioned herein the genetic determinant of the invention that leads to the ToLCNDV resistance comprises more than one QTL and that those QTLs are located on separate chromosomes of the *Cucumis melo* genome. The combination of two resistance-conferring QTLs, QTL-1 and QTL-2 leads to a higher level of resistance to ToLCNDV.

Deposit number NCIMB 42557 or progeny seed thereof, can suitably be used as a source to introgress QTL-1, located on LG 5, and/or QTL-2, located on LG 9 into a *Cucumis melo.* SNP analysis and interval mapping of a large number of SNP markers in the genome of crosses between NCIMB 42557 and commercial melon cultivars, the ToLCNDV-resistance-conferring QTL-1 located on linkage group 5 between physical positions 24,008,941 bp and 24,895,385 bp (on the basis of the melon genome version CM3.5), was found to be linked with at least one of the markers selected from: CM_0205 comprising the SEQ ID NO: 1, MEL_SNP_2224 comprising the SEQ ID NO: 3, MEL_SNP_2208 comprising the SEQ ID NO: 2, or combinations thereof. The ToLCNDV-resistance-conferring QTL-1 spanning about 46.9 to 52.7 cM. The markers identified herein may be used in various aspects of the invention as will now be illustrated. Preferably, the ToLCNDV-resistance-conferring QTL-1 is associated with the marker MEL_SNP_2208 comprising the SEQ ID NO: 2, and further can also be associated with the markers CM_0205 comprising the SEQ ID NO: 1, MEL_SNP_2224 comprising the SEQ ID NO: 3, or combinations thereof. The ToLCNDV-resistance-conferring QTL-2 located on linking group 9 between physical positions 22,847,688 bp and 23, 556,554 bp (on the basis of the melon genome version CM3.5), was found to be linked with at least one of the markers selected from: MEL_SNP_4114 comprising the SEQ ID NO: 5, MEL_SNP_4105 comprising the SEQ ID NO: 4, SFMe066 comprising the SEQ ID NO: 6, or combinations thereof. Preferably, the ToLCNDV-resistance-conferring QTL-2 is associated with the marker MEL_SNP_4114 comprising the SEQ ID NO: 5 and further can also be associated with the markers MEL_SNP_4105 comprising the SEQ ID NO: 4, SFMe066 comprising the SEQ ID NO: 6, or combinations thereof. The ToLCNDV-resistance-conferring QTL-2 spanning about 66.1 to 70.1 cM.

Aspects of the invention are not being limited to the use of the markers identified herein. It is stressed that the aspects may also make use of markers not explicitly disclosed herein or even yet to be identified.

The present inventors have now discovered two QTLs for begomovirus-resistance, preferably for ToLCNDV-resistance in melon that is carried into offspring plants. The inventors made this discovery by observing that the presence of a string of contiguous genomic markers belonging to linkage group 5, and further to linkage group 9, i.e. on two different chromosomes in the genome of melon correlated to the presence of a particular phenotypic trait that affected the occurrence of disease symptoms after exposure to an natural infection of ToLCNDV and they showed that this genomic region was inherited according to normal Mendelian laws of inheritance. In general, a QTL may span a region of several million bases. Therefore, providing the complete sequence information for the QTL is practically unfeasible but also unnecessary, as the way in which the QTL is first detected-through the observed correlation between the presence of a string of contiguous genomic markers and the presence of a particular phenotypic trait-allows one to trace amongst offspring plants those plants that have the genetic potential for exhibiting a particular phenotypic trait. By providing a non-limiting list of markers, the present invention thus provides for the effective utility of the QTL in a breeding program.

The QTL as described herein was found by crossing *C. melo var. momordica* (NCIMB 42557) with one elite line of *Cucumis melo* var. *Cantalupensis* and screening for resistance to ToLCNDV using a conventional bioassay (field-test under natural ToLCNDV pressure). LOD scores for the SNP markers significantly higher than 2.8 were considered to indicate the QTL.

A marker is specific for a particular line of breed. Thus, a specific trait is associated with a particular marker. The markers as indicated in the present application do not only indicate the location of the QTL, they also correlate to the presence of the specific phenotypic trait in a plant. It is important to note that the contiguous genomic markers that indicate the location of the QTL on the genome are in principal arbitrary or non-limiting. In general, the location of a QTL is indicated by a contiguous string of markers that exhibit statistical correlation to the phenotypic trait. Once a marker is found outside that string (i.e. one that has a LOD-score below a certain threshold, indicating that the marker is so remote that recombination in the region between that marker and the QTL occurs so frequently that the presence of the marker does not correlate in a statistically significant manner to the presence of the phenotype) the boundaries of the QTL are set. Thus, it is also possible to indicate the location of the QTL by other markers located within that specified region.

It is further important to note that the contiguous genomic markers can also be used to indicate the presence of the QTL (and thus of the phenotype) in an individual plant, i.e. they can be used in MAS procedures. In principle, the number of potentially useful markers is limited but may be very large, and the skilled person may easily identify additional markers to those mentioned in the present application (See Table 1). Any marker that is linked to the QTL, e.g. falling within the physically boundaries of the genomic region spanned by the markers having established LOD scores above a certain threshold thereby indicating that no or very little recombination between the marker and the QTL occurs in crosses; as well as any marker in linkage disequilibrium to the QTL; as well as markers that represent the actual causal mutations within the QTL, may be used in MAS procedures. This means that the markers identified in the application as associated to the QTL-1, such as the marker MEL_SNP_2208 comprising the SEQ ID NO: 2, CM_0205 comprising the SEQ ID NO: 1, MEL_SNP_2224 comprising the SEQ ID NO: 3, or any combinations thereof, are mere examples of markers suitable for use in MAS procedures. Additionally, the markers identified in the application as associated to the QTL-2, such as the marker MEL_SNP_4114 comprising the SEQ ID NO: 5, MEL_SNP_4105 comprising the SEQ ID NO: 4, SFMe066 comprising the SEQ ID NO: 6, or any combinations thereof, are also mere examples of markers suitable for use in MAS procedures. Moreover, when the QTL, or the specific trait-conferring part thereof, is introgressed into another genetic background (i.e. into the genome of another plant variety), then some markers may no longer be found in the offspring although the trait is present therein, indicating that such markers are outside the genomic region that represents the specific trait-conferring part of the QTL in the original parent line only and that the new genetic background has a different genomic organisation.

The present invention also refers to a part of the ToLCNDV resistant plant of the present invention. In a preferred embodiment, the part of the ToLCNDV resistant plant of the present invention are selected from the list consisting of: single cells and cell tissues such as plant cells that are intact in plants, cell clumps and tissue cultures from which melon plants of the present invention can be regenerated. Examples of plant parts include, but are not limited to, single cells and tissues from pollen, ovules, leaves, embryos, roots, root tips, anthers, flowers, fruits, stems shoots, and seeds; as well as pollen, ovules, leaves, embryos, roots, root tips, anthers, flowers, fruits, stems, shoots, scions, rootstocks, seeds, protoplasts, calli, and the like. In a preferred embodiment, the preferred plant parts are seeds or fruits. The part of the ToLCNDV resistant plant of the present invention harbour is their genetic constitution the genetic information that lead to the resistance characteristics that define the melon plants of the invention carries the genetic information that leads to phenotypic expression of said traits or QTLs.

The present invention also relates to the progeny of the plants or plants parts of the present invention. Such progeny can in itself be plants or plant parts as mentioned herein.

As used herein the term "progeny" is intended to mean the first and all further descendants from a cross with a plant of the invention that comprises a genetic determinant that leads to ToLCNDV resistance. Progeny of the invention are descendants of any cross with a plant of the invention that carries the trait that leads to ToLCNDV resistance. In one embodiment, progeny plants of the invention carry one or more of the QTL-1 and QTL-2 that constitute the genetic determinant of the invention that leads to resistance to ToLCNDV.

The present invention also refers to a ToLCNDV-resistance-conferring QTL-1 located on linkage group 5 between physical positions 24,008,941 bp and 24,895,385 bp (on the basis of the melon genome version CM3.5), of NCIMB 42557, and wherein the ToLCNDV-resistance-conferring QTL-1 is detectable by a molecular marker assay which detects at least the SNP marker MEL_SNP_2208 comprising the SEQ ID NO: 2. In a preferred embodiment, the QTL-1 is further detectable by the SNP markers CM_0205 comprising the SEQ ID NO: 1, MEL_SNP_2224 comprising the SEQ ID NO: 3, or any combinations thereof.

Additionally, the present invention also refers to a ToLCNDV-resistance-conferring QTL-2 located on linkage group (LG) 9 between physical positions 22,847,688 bp and 23,556,554 bp (on the basis of the melon genome version CM3.5), or a ToLCNDV-resistant conferring part thereof, of NCIMB 42557, and wherein the ToLCNDV-resistance-conferring QTL-2 or a ToLCNDV-resistant conferring part thereof is detectable by a molecular marker assay which detects at least the SNP marker MEL_SNP_4114 comprising the SEQ ID NO: 5. In a preferred embodiment, the QTL-2 is further detectable by the SNP markers MEL_SNP_4105 comprising the SEQ ID NO: 4, SFMe066 comprising the SEQ ID NO: 6, or any combinations thereof.

The nucleic acid sequence of the two QTLs of the present invention may be determined by methods known to the skilled person. For instance, a nucleic acid sequence comprising at least one of said QTL, preferably the two QTLs of the present invention, or a resistance-conferring part thereof, may be isolated from a ToLCNDV-resistant donor plant by fragmenting the genome of said plant and selecting those fragments harboring one or more markers indicative of said QTLs. Subsequently, or alternatively, the marker sequences (or parts thereof) indicative of said QTLs may be used as (PCR) amplification primers, in order to amplify a nucleic acid sequence comprising said QTLs from a genomic nucleic acid sample or a genome fragment obtained from said plant. The amplified sequence may then be purified in order to obtain the isolated QTLs. The nucleotide sequence of the QTLs, and/or of any additional markers comprised therein, may then be obtained by standard sequencing methods.

The present invention therefore also discloses a molecular marker, preferably a molecular SNP marker, present in *Cucumis melo* genome, which molecular marker is genetically linked to QTL that confers resistance to ToLCNDV in *Cucumis melo,* and which molecular marker is characterized by any of the SEQ ID NO: 1-6 and wherein the QTL is preferably the QTL-1 and/or the QTL-2. In a preferred embodiment, the molecular SNP markers of SEQ ID NOs: 1 to 3 are linked to QTL-1 and the molecular SNPs marker of SEQ ID NOs: 4 to 5 are linked to QTL-2.

The present invention therefore also relates to an isolated nucleic acid, preferably DNA, sequence that comprises a QTL of the present invention, or a begomovirus-resistance-conferring part thereof, preferably a ToLCNDV-resistance-conferring part thereof. Thus, the markers that pinpoint the QTLs described herein may be used for the identification, isolation and purification of one or more genes from melon that encode for begomovirus resistance, preferably for a ToLCNDV resistance.

The nucleotide sequence of the QTLs of the present invention may for instance also be resolved by determining the nucleotide sequence of one or more markers associated with said QTLs and designing internal primers for said marker sequences that may then be used to further determine the sequence the QTLs outside of said marker sequences. For instance the nucleotide sequence of the markers identified herein may be obtained by isolating said markers from the electrophoresis gel used in the determination of the presence of said markers in the genome of a subject plant, and determining the nucleotide sequence of said markers by for instance dideoxy chain terminating methods, well known in the art.

Another embodiment of the present invention refers to a method for producing a plant of the present invention comprising introgressing into a begomovirus-susceptible melon plant, preferably into a ToLCNDV-susceptible melon plant the at least one begomovirus-resistance-conferring QTL, preferably, the ToLCNDV-resistance-conferring QTL-1 from NCIMB 42557, and more preferably the ToLCNDV-resistance-conferring QTL-1 and QTL-2 from NCIMB 42557, and involving MAS procedures, is not limited to the use of markers that are provided herein for the sole purpose of (roughly) indicated the location of the QTL-1 and/or QTL-2 in the chromosomes 5 and 9, respectively. The skilled person knows that other markers may provide at least equal utility in such MAS procedures.

In embodiments of such methods for detecting the presence of a QTL in a suspected ToLCNDV-resistant melon plant, the method may also comprise the steps of providing a oligonucleotide or polynucleotide capable of hybridizing under stringent hybridization conditions to a nucleic acid sequence of a marker linked to said QTL, preferably selected from the markers identified herein as being linked to said QTL, contacting said oligonucleotide or polynucleotide with a genomic nucleic acid of a suspected ToLCNDV-resistant melon plant, and determining the presence of specific hybridization of said oligonucleotide or polynucleotide to said genomic nucleic acid. Preferably said method is performed on a nucleic acid sample obtained from said suspected ToLCNDV-resistant melon plant, although *in situ* hybridization methods may also be employed. Alternatively, and in a more preferred embodiment, the skilled person may, once the nucleotide sequence of the QTL has been determined, design specific hybridization probes or oligonucleotides capable of hybridizing under stringent hybridization conditions to the nucleic acid sequence of said QTL and may use such hybridization probes in methods for detecting the presence of a QTL of the invention in a suspected ToLCNDV-resistant melon plant.

Examples of probes or oligonucleotides capable of hybridizing under stringent hybridization conditions to the nucleic acid sequence of the QTLs include, but are not limited to the probes or oligonucleotides shown in Table 2.

The terms "stringency" or "stringent hybridization conditions" refers to conditions under which a probe or polynucleotide will hybridize to its target subsequence, typically in a complex mixture of nucleic acids, but to essentially no other sequences. Stringent conditions are sequence-dependent and will be different in different circumstances. Longer sequences hybridize specifically at higher temperatures. An extensive guide to the hybridization of nucleic acids is found in Tijssen (Tijssen, 1993). Generally, stringent conditions are selected to be about 5-100°C lower than the thermal melting point (Tm) for the specific sequence at a defined ionic strength pH. The Tm is the temperature (under defined ionic strength, pH, and nucleic acid concentration) at which 50% of the probes complementary to the target hybridize to the target sequence at equilibrium (as the target sequences are present in excess, at Tm, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 300°C for short probes (e.g., 10 to 50 nucleotides) and at least about 60°C for long probes (e.g., greater than 50 nucleotides). Stringent conditions may also be achieved with the addition of destabilizing agents such as formamide. For selective or specific hybridization, a positive signal is at least two times background, preferably 10 times background hybridization. Exemplary stringent hybridization conditions are often: 50% formamide, 5xSSC, and 1% SDS, incubating at 42°C, or, 5xSSC, 1% SDS, incubating at 65°C, with wash in 0.2xSSC, and 0.1% SDS at 65°C. For PCR, a temperature of about 36°C is typical for low stringency amplification, although annealing temperatures may vary between about 32°C and 48°C depending on primer length. Additional guidelines for determining hybridization parameters are provided in numerous references, e.g. Current Protocols in Molecular Biology, eds. Ausubel, et al. 1995).

### Production of begomovirus-resistant melon plants, preferable a ToLCNDV-resistant melon plants by non-transgenic methods.

In an alternative embodiment for producing a begomovirus-resistant melon plant, preferably a ToLCNDV-resistant melon plant, protoplast fusion can be used for the transfer of nucleic acids from a donor plant to a recipient plant. Protoplast fusion is an induced or spontaneous union, such as a somatic hybridization, between two or more protoplasts (cells of which the cell walls are removed by enzymatic treatment) to produce a single bi- or multi-nucleate cell. The fused cell that may even be obtained with plant species that cannot be interbreeded in nature, is tissue cultured into a hybrid plant exhibiting the desirable combination of traits. More specifically, a first protoplast can be obtained from a melon plant or other plant line that exhibits resistance to infection by begomovirus, preferably by a ToLCNDV. A second protoplast can be obtained from a second melon or other plant line, preferably a melon line that comprises commercially valuable characteristics, such as, but not limited to disease resistance, insect resistance, valuable fruit characteristics, etc. The protoplasts are then fused using traditional protoplast fusion procedures, which are known in the art.

Alternatively, embryo rescue may be employed in the transfer of a nucleic acid comprising one or more QTLs as described herein from a donor plant to a recipient plant. Embryo rescue can be used as a procedure to isolate embryo's from crosses wherein plants fail to produce viable seed. In this process, the fertilized ovary or immature seed of a plant is tissue cultured to create new plants (Pierik, 1999).

The present invention also relates to a method of producing a begomovirus-resistant melon plant, preferably a ToLCNDV-resistant melon plant comprising the steps of performing a method for detecting the presence of at least one QTL associated with resistance to ToLCNDV in a donor melon plant according to invention as described above, and transferring a nucleic acid sequence comprising at least one QTL thus detected, or a ToLCNDV-resistance-conferring part thereof, from said donor plant to a ToLCNDV-susceptible recipient melon plant. The transfer of said nucleic acid sequence may be performed by any of the methods previously described herein.

A preferred embodiment of such a method comprises the transfer by introgression of said nucleic acid sequence from a ToLCNDV-resistant donor melon plant into a ToLCNDV-susceptible recipient melon plant by crossing said plants. This transfer may thus suitably be accomplished by using traditional breeding techniques. QTLs are preferably introgressed into commercial melon lines by using MAS. Marker-assisted breeding or marker- assisted selection involves the use of one or more of the molecular markers for the identification and selection of those offspring plants that contain one or more of the genes that encode for the desired trait. In the present instance, such identification and selection is based on selection of QTLs of the present invention or markers associated therewith. MAS can also be used to develop near-isogenic lines (NIL) harboring the QTLs of interest, allowing a more detailed study of each QTL effect and is also an effective method for development of backcross inbred line (BIL) populations (see e.g. Nesbitt et al., 2001; van Berloo et al., 2001). Melon plants developed according to this embodiment can advantageously derive a majority of their traits from the recipient plant, and derive ToLCNDV-resistance from the donor plant. As discussed briefly above, traditional breeding techniques can be used to introgress a nucleic acid sequence encoding for ToLCNDV-resistance into a ToLCNDV-susceptible recipient melon plant. In one method, which is referred to as pedigree breeding, a donor melon plant that exhibits resistance to ToLCNDV and comprising a nucleic acid sequence encoding for ToLCNDV-resistance is crossed with a ToLCNDV-susceptible recipient melon plant that preferably exhibits commercially desirable characteristics, such as, but not limited to, disease resistance, insect resistance, valuable fruit characteristics, etc. The resulting plant population (representing the F1 hybrids) is then self-pollinated and set seeds (F2 seeds). The F2 plants grown from the F2 seeds are then screened for resistance to ToLCNDV. The population can be screened in a number of different ways.

First, the population can be screened using a traditional disease screen. Such disease screens are known in the art. Preferably a quantitative bioassay is used. Second, marker-assisted selection can be performed using one or more of the hereinbefore-described molecular markers to identify those progeny that comprise a nucleic acid sequence encoding for TOLCNDV-resistance. Other methods, referred to hereinabove by methods for detecting the presence of a QTL may be used. Also, marker-assisted selection can be used to confirm the results obtained from the quantitative bioassays, and therefore, several methods may also be used in combination.

Inbred ToLCNDV-resistant melon plant lines can be developed using the techniques of recurrent selection and backcrossing, selfing and/or dihaploids or any other technique used to make parental lines. In a method of recurrent selection and backcrossing, ToLCNDV-resistance can be introgressed into a target recipient plant (the recurrent parent) by crossing the recurrent parent with a first donor plant, which differs from the recurrent parent and is referred to herein as the "non-recurrent parent". The recurrent parent is a plant that is non-resistant or has a low level of resistance to ToLCNDV and possesses commercially desirable characteristics, such as, but not limited to (additional) disease resistance, insect resistance, valuable fruit characteristics, etc. The non-recurrent parent exhibits ToLCNDV resistance and comprises a nucleic acid sequence that encodes for ToLCNDV -resistance. The non-recurrent parent can be any plant variety or inbred line that is cross-fertile with the recurrent parent. The progeny resulting from a cross between the recurrent parent and non-recurrent parent are backcrossed to the recurrent parent. The resulting plant population is then screened for the desired characteristics, which screening may occur in a number of different ways. For instance, the population can be screened using phenotypic pathology screens or quantitative bioassays as known in the art. Alternatively, instead of using bioassays, MAS can be performed using one or more of the hereinbefore described molecular markers, hybridization probes or polynucleotides to identify those progeny that comprise a nucleic acid sequence encoding for ToLCNDV-resistance. Also, MAS can be used to confirm the results obtained from the quantitative bioassays. In case the QTL is located on a recessive locus, this means that the resistance gene cannot be screened in an F1 or BC1 population by using phenotypic screens such as resistance bioassays. The markers defined herein are therefore ultimately suitable to select proper offspring plants by genotypic screening. Following screening, the F1 hybrid plants that exhibit a ToLCNDV-resistant phenotype or, more preferably, genotype and thus comprise the requisite nucleic acid sequence encoding for ToLCNDV-resistance are then selected and backcrossed to the recurrent parent for a number of generations in order to allow for the melon plant to become increasingly inbred. This process can be performed for two to five or more generations. In principle the progeny resulting from the process of crossing the recurrent parent with the ToLCNDV-resistance non-recurrent parent are heterozygous for one or more genes that encode for ToLCNDV-resistance. It should be brought into mind that, for instance when introgressing a recessive locus the resistant phenotype will only occur in offspring plants under conditions wherein homozygous plants can be formed.

In general, a method of introducing a desired trait into a hybrid melon variety comprises the steps of:
a) crossing an inbred melon parent with another melon plant that comprises one or more desired traits (QTLs of the present invention), to produce F1 progeny plants, wherein the desired trait is selected from the group consisting of ToLCNDV-resistance, preferably, QTL-1 and/or QTL-2;
b) selecting said F1 progeny plants that have the desired trait to produce selected F1 progeny plants, preferably using molecular markers as defined herein;
c) backcrossing the selected progeny plants with said inbred melon parent plant to produce backcross progeny plants;
d) selecting for backcross progeny plants that have the desired trait and morphological and physiological characteristics of said inbred melon parent plant, wherein said selection comprises the isolation of genomic DNA and testing said DNA for the presence of at least one molecular marker for QTLs of the present invention, preferably as described herein;
e) repeating steps (c) and (d) two or more times in succession to produce selected third or higher backcross progeny plants;
f) optionally selfing selected backcross progeny in order to identify homozygous plants; and
g) crossing at least one of said backcross progeny or selfed plants with another inbred melon parent plant to generate a hybrid melon variety with the desired trait and all of the morphological and physiological characteristics of hybrid melon variety when grown in the same environmental conditions.

As indicated, the last backcross generation may be selfed in order to provide for homozygous pure breeding (inbred) progeny for ToLCNDV-resistance. Thus, the result of recurrent selection, backcrossing and selfing is the production of lines that are genetically homogenous for the genes associated with ToLCNDV-resistance as well as other genes associated with traits of commercial interest.

### Begomovirus, preferably ToLCNDV-resistant melon plants and seeds.

The goal of plant breeding is to combine in a single variety or hybrid various desirable traits or QTLs. For commercial crops, these traits may include resistance to diseases and insects, tolerance to heat and drought, reducing the time to crop maturity, greater yield, and better agronomic quality. Uniformity of plant characteristics such as germination and stand establishment, growth rate, maturity, and plant height may also be of importance.

Commercial crops are bred through techniques that take advantage of the plant's method of pollination. A plant is self-pollinated if pollen from one flower is transferred to the same or another flower of the same plant. A plant is sib-pollinated when individuals within the same family or line are used for pollination. A plant is cross-pollinated if the pollen comes from a flower on a different plant from a different family or line. Plants that have been self-pollinated and selected for type for many generations become homozygous at almost all gene loci and produce a uniform population of true breeding progeny. A cross between two different homozygous lines produces a uniform population of hybrid plants that may be heterozygous for many gene loci. A cross of two plants each heterozygous at a number of gene loci will produce a population of heterogeneous plants that differ genetically and will not be uniform.

The development of a hybrid melon variety in a melon plant breeding program involves three steps: (1) the selection of plants from various germplasm pools for initial breeding crosses; (2) the selfing of the selected plants from the breeding crosses for several generations to produce a series of inbred lines, which, individually breed true and are highly uniform; and (3) crossing a selected inbred line with an unrelated inbred line to produce the hybrid progeny (F1). After a sufficient amount of inbreeding successive filial generations will merely serve to increase seed of the developed inbred. Preferably, an inbred line should comprise homozygous alleles at about 95% or more of its loci.

An important consequence of the homozygosity and homogeneity of the inbred lines is that the hybrid created by crossing a defined pair of inbreds will always be the same. Once the inbreds that create a superior hybrid have been identified, a continual supply of the hybrid seed can be produced using these inbred parents and the hybrid melon plants can then be generated from this hybrid seed supply.

A ToLCNDV-resistant melon plant, or a part thereof, obtainable by a method of the invention is another aspect of the present invention. Therefore, the present invention also relates to a ToLCNDV-resistant melon plant, or part thereof, comprising the QTLs in any configuration as described in detail above. The ToLCNDV-resistant melon plants of the present invention can be of any genetic type such as inbred, hybrid, haploid, dihaploid or transgenic. Further, the plants of the present invention may be heterozygous or homozygous for the resistance traits, preferably homozygous. Although the QTLs of the present invention, as well as resistance-conferring parts thereof may be transferred to any plant in order to provide for a ToLCNDV-resistant plant, the methods and plants of the invention are preferably related to plants of the species *Cucumis melo.* The ToLCNDV-resistant inbred melon lines described herein can be used in additional crossings to create ToLCNDV-resistant hybrid plants. For example, a first ToLCNDV-resistant inbred melon plant of the invention can be crossed with a second inbred melon plant possessing commercially desirable traits such as, but not limited to, disease resistance, insect resistance, desirable fruit characteristics, etc. This second inbred melon line may or may not be ToLCNDV-resistant.

Another aspect of the present invention relates to a method of producing seeds that can be grown into ToLCNDV-resistant melon plants. In one embodiment, the method comprises the steps of providing a ToLCNDV-resistant melon plant of the invention, crossing said ToLCNDV-resistant plant with another melon plant, and collecting seeds resulting from said cross, which when planted, produce ToLCNDV-resistant melon plants.

In another embodiment, the method comprises the steps of providing a ToLCNDV-resistant melon plant of the invention, crossing said ToLCNDV-resistant plant with a melon plant, collecting seeds resulting from said cross, regenerating said seeds into plants, selecting ToLCNDV-resistant plants by any of the methods described herein, self-pollinating the selected plants for a sufficient number of generations to obtain plants that are fixed for an allele that confers ToLCNDV-resistance in the plants, backcrossing the plants thus produced with melon plants having desirable phenotypic traits for a sufficient number of generations to obtain melon plants that are ToLCNDV-resistant and have desirable phenotypic traits, and collecting the seeds produced from the plants resulting from the last backcross, which when planted, produce melon plants which are ToLCNDV-resistant.

A further embodiment of the invention is a method of identifying a ToLCNDV-resistant melon plant of the invention, comprising:
a) providing a population of cultivated *C. melo* plants,
b) screening said population using a molecular marker assay which detects at least one SNP marker selected from the group consisting of: SNP marker MEL_SNP_2208 comprising the SEQ ID NO: 2; CM_0205 comprising the SEQ ID NO: 1, MEL_SNP_2224 comprising the SEQ ID NO: 3, MEL_SNP_4114 comprising the SEQ ID NO: 5, MEL_SNP_4105 comprising the SEQ ID NO: 4, SFMe066 comprising the SEQ ID NO: 6, or any combinations thereof; and
c) identifying and/or selecting a plant comprising at least one of the SNP markers selected from the group consisting of: SNP marker MEL_SNP_2208 comprising the SEQ ID NO: 2; CM_0205 comprising the SEQ ID NO: 1, MEL_SNP_2224 comprising the SEQ ID NO: 3, MEL_SNP_4114 comprising the SEQ ID NO: 5, MEL_SNP_4105 comprising the SEQ ID NO: 4, SFMe066 comprising the SEQ ID NO: 6, or any combinations thereof.

In a preferred embodiment, the method of identifying the ToLCNDV-resistant melon plant of the invention it is characterized by the SNP marker MEL_SNP_2208 comprising the SEQ ID NO: 2 is detected, preferably, by the primers of SEQ ID NO: 10, 11 and 12; SNP marker CM_0205 comprising the SEQ ID NO: 1 is detected, preferably, by the primers of SEQ ID NO: 7, 8 and 9 and the SNP marker MEL_SNP_2224 comprising the SEQ ID NO: 3 is detected, preferably, by the primers of SEQ ID NO: 13, 14 and 15, SNP marker MEL_SNP_4114 comprising the SEQ ID NO: 5 is detected, preferably, by the primers of SEQ ID NO: 19, 20 and 21; SNP marker MEL_SNP_4105 comprising the SEQ ID NO: 4 is detected, preferably, by the primers of SEQ ID NO: 16, 17 and 18; and the SNP marker SFMe066 comprising the SEQ ID NO: 6 is detected, preferably, by the primers of SEQ ID NO: 22, 23 and 24.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, drawings and sequence listing are provided by way of illustration and are not intended to be limiting of the present invention.

### EXAMPLES

The following examples are offered to illustrate, but not to limit, the claimed invention. It is understood that the examples and embodiments described herein are for illustrative purposes only, and persons skilled in the art will recognize various reagents or parameters that can be altered without departing from the spirit of the invention or the scope of the appended claims.

### Example 1. ToLCNDV-resistant plants. Analysis of resistance on chromosomes 5 and 9 of NCIMB 42557.

The mapping genetic population of 110 Bc1S1 families was generated after one generation of backcrossing and one generation of selfing. First, the F1 generation was produced by crossing the resistant plants whose seed was deposited with the NCIMB under accession number NCIMB 42557 to the recurrent parental line A0803. The recurrent parental line A0803 is a muskmelon *(Cucumis melo var. Cantalupens)* with orange flesh, moderate aroma, average Brix around 12 degrees and appropriate flavor. The F1 plants were grown and crossed to the recurrent line A0803 in order to generate the first backcross plants named as Bc1 population. Next, 110 Bc1 plants were grown and each of them was labeled and selfed in order to generate Bc1S1 progeny per each family.

DNA extraction and molecular marker analysis was performed by the isolation of the genomic DNA from young leaf tissue of the both parents (NCIMB 42557 and A0803 line), the F1 generation and the 110 Bc1 plants using a semi-automated Biosprint 96 (Qiagen) DNA extraction platform following a proven MagAttract magnetic particle technology as described by the manufacturer. Additionally, the molecular analysis was performed using proprietary SNPs markers that had been previously developed in-house. The position of each SNP in the genome of melon was identified after BLASTING each DNA fragment against the melon genome database www.melonomics.net, on the basis of the melon genome version CM3.5. First, 350 SNPs that were homogeneously distributed in the twelve chromosomes of melon were used to genotype only the two parental lines, the NCIMB 42557 and the A0803. The aim of this genotyping was to identify the ones that were polymorphic between the donor (NCIMB 42557) and the recurrent parent line (A0803). One hundred SNP markers were found polymorphic and were used to genotype the 110 Bc1 plants.

KBioscience Competitive Allele-Specific PCR genotyping system (KASPAR™) assays were developed for the SNPs following the instructions provided by the manufacture (LGC, Teddington, Middlesex, TW11 0LY, UK). More technical information about the KASPAR™ system, including a guide of solutions to common problems, is obtainable from LGC Genomics (LGC, KASP genotyping chemistry User guide and manual), or Semagn K, et al. 2013. Molecular Breeding 33 (1): 1-14.

In order to obtain the genetic map of the ToLCNDV-resistant plants of the present invention, linkage analysis and segregation distortion tests (P≤0.05) were performed using JoinMap 4.0 software analysis (Van Ooijen et al., 2006). Markers were assigned to linkage groups with a LOD score of 3. The linkage map calculations were done using all pairwise recombination estimates lower than 0.49 and a LOD score higher than 0.01, and applying the Kosambi mapping function (Kosambi et al., 1944). Individuals and markers with more than 10% missing values were removed from the original molecular data set. Also markers with a severe segregation distortion (P≤ 0.005) were excluded. After a preliminary map analysis, improbable genotypes, including double recombination events (singletons), markers with suspected linkages with other markers and redundant markers clustered at the same position were removed. To check the reliability of the obtained map, the individual linkage group _{X}2 was inspected.

In order to analyze and identify the ToLCNDV-resistant plants of the present invention, seeds of the 110 Bc1 S1 families and the two parents (NCIMB 42557 and A0803 line) were sown in pots of 3x3 cm and were incubated at 24 °C growth chambers during 48 hours in late August. Then, they were placed in the greenhouse until the seedlings reach the appropriate size for transplantation. Eight to twelve plants per family were sown in a randomized complete block design. Appropriate sensible controls were sown in replication in order to certify the natural incidence of the virus. The greenhouse was located in the region of Almeria where the extensive virus incidence was recorded. The plants were placed in a distance of 40 cm between them, resulting in density of 1.4 plants/m². Water and nutrients were administered in the plants depending on weather conditions, temperature, humidity, radiation, etc. as well as the growth stage of the crop (vegetative growth, ripening, etc.). To promote the natural ToLCNDV virus transmission and infection, the greenhouse windows were left open to allow the entrance of the whitefly *(Bemisia tabaci)* from outside. No application of any chemical agent was done in order to allow the survival and the multiplication of the whitefly.

Phenotyping was performed on the basis of the typical symptoms that are caused by ToLCNDV and are as described below: stunting plants, yellow leaf mosaic, curling leaves and vein swelling. On cucurbit fruit, rough skin and longitudinal cracking can be observed. If the virus infection occurs at an early stage, affected plants are severely stunted and fruit production is largely deteriorated, if not vanished at all. Six different scorings were performed in the months of October and November. The plants that showed any of the above symptoms were classified as susceptible. Finally, the Disease Index (DI) was calculated as the ratio of susceptible plants to total number of plants grown per family. The Disease Index was used for the subsequent statistical analysis and the detection of loci involved in the resistance.

For the QTL detection, mapping and maker development, first, phenotypic data descriptive statistics were calculated using the package R (https://www.r-project.org/). The trait distribution was graphically demonstrated in order to assess if the genetic model that explains the observed variation, follows a quantitative or mendelian inheritance. The previously obtained F2 refined linkage map was used for QTL identification. QTL analysis was performed on entry means from each family. Kruskal-Wallis single-marker analysis (non-parametric test), as well as for both interval mapping (Lander et al., 1989) and multiple-QTL mapping (MOM) (Jansen et al., 1994) were performed using MapQTL version 4.0 (Van Ooijen et al., 2002). A backward elimination procedure was applied to select cofactors significantly associated with each trait at P < 0.02 to be used in MOM. Genome-wide threshold values (P < 0.05) for declaring the presence of QTL were estimated from 10,000 permutations of each phenotypic trait (Churchill et al., 1994). Approximate confidence intervals for each LOD peak were constructed using the one-LOD and the two-LOD rule with the confidence interval being defined by all those values falling within one or two LOD score of the maximum value. The R² value, representing the percentage of the phenotypic variance explained by the marker genotype at the QTL, was taken from the peak QTL position as estimated by MapQTL. Additive and dominance effects for detected QTL were estimated using the MOM procedure. Graphs depicting the LOD score against the genetic map were drawn using MapChart version 2.2 software (Voorrips et al., 2002). These graphs show also the confidence interval of the QTLs in cM as estimated by the one-LOD rule: the confidence interval being defined by all those values falling within one LOD score of the maximum value.

### Identification of QTLs associated with ToLCNDV resistance in NCIMB 42557.

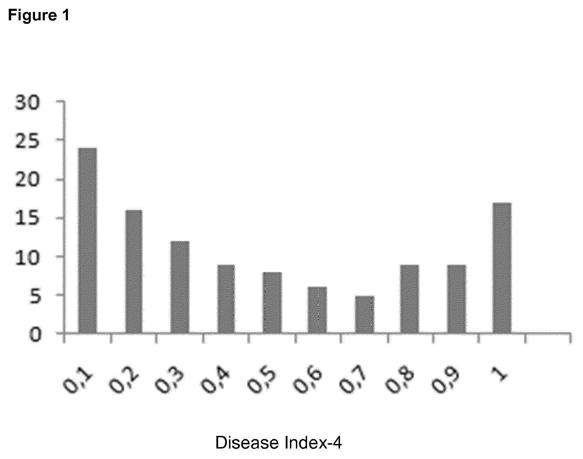

The frequency distribution of the Disease Index for the phenotyping score 4 is skewed towards the extremes and does not follow a typical quantitative distribution (Fig.1). This implies that the disease resistance is more or less of qualitative nature and with one or two responsible genes of dominant type.

Two regions or QTLs have been identified for NCIMB 42557 that contributed to the resistance were located, positioned on two separate chromosomes. Molecular SNP markers that correlated most closely to the QTLs are presented in Table 1. For positioning the QTL, the publicly available map of the *Cucumis melo* (www.melonomics.net), on the basis of the melon genome version CM 3.5, was used as reference for all positions mentioned herein. Moreover, the positions of the QTLs were linked to different SNP markers which positions in the physical map of the melon genome were identified by BLAST. For each SNP marker two allele-specific forward (A1 and A2) and one common reverse (C1) primers were developed, as indicated in Table 2 (all sequences are given in 5 'to 3 'direction).

A first QTL (named as QTL-1) which explained 35% of the variation was located on chromosome 5, having a LOD score of 9.97. These data explain the qualitative nature of the genetic resistance. The LOD score is graphed against the genetic map in Figure 2. The position of the QTL-1 was determined to be between the physical positions 24,008,941 bp to 24,895,385 bp (on the basis of the melon genome version CM3.5). In the BC1S1 population and subsequent progeny this QTL-1 was linked most closely to a SNP marker on position 24,242,219 bp, the sequence of which is found in Table 1 as MEL_SNP_2208 comprising the SEQ ID NO: 2 The borders of the QTL-1 region were identified by the presence of markers CM_0205 comprising the SEQ ID NO: 1 and MEL_SNP_2224 comprising the SEQ ID NO: 3. The preferred marker MEL_SNP2208 comprising the SEQ ID NO: 2 is situated in 0.2 cM from the predicted location of the locus of chromosome 5. In the Figure 2, the confidence interval of 46.9-52.7 cM, preferably 49.7 cM of the QTL using the one-LOD rule is depicted.

A second QTL (named as QTL-2) which explained 10% of the variation was located on chromosome 9, having a LOD score of 3.02. The LOD score is graphed against the genetic map in Figure 3. The position of the QTL-2 was determined to be between the physical positions 22,847,688 bp to 23,556,554 bp on the basis of the genome version CM3.5. In the BC1S1 population and subsequent progeny this QTL-2 was linked most closely to a SNP marker on position 23,538,169 bp, the sequence of which is found in Table 1 as MEL_SNP _4114 comprising the SEQ ID NO: 5 The borders of the QTL-2 region were identified by the presence of markers MEL_SNP_4105 comprising the SEQ ID NO: 4 and SFMe066 comprising the SEQ ID NO: 6. The preferred marker MEL_SNP_4114 comprising the SEQ ID NO: 5. In the Figure 3, the confidence interval of 66.1-70.1 cM, preferably 68.3 cM of the QTL using the one-LOD rule is depicted.

SEQ ID NO: 1 comprises the presence of a SNP from nucleotide C to T at 61^{th} position (bold) in the nucleotide sequence shown in Table 1.

SEQ ID NO: 2 comprises the presence of a SNP from nucleotide G to T at 51^{th} position (bold) in the nucleotide sequence shown in Table 1.

SEQ ID NO: 3 comprises the presence of a SNP from nucleotide T to C at 51^{th} position (bold) in the nucleotide sequence shown in Table 1.

SEQ ID NO: 4 comprises the presence of a SNP from nucleotide C to T at 51^{th} position (bold) in the nucleotide sequence shown in Table 1.

SEQ ID NO: 5 comprises the presence of a SNP from nucleotide G to T at 51^{th} position (bold) in the nucleotide sequence shown in Table 1.

SEQ ID NO: 6 comprises the presence of a SNP from nucleotide A to G at 101^{st} position (bold) in the nucleotide sequence shown in Table 1.

**Table 1. Molecular SNP markers that in deposit number NCIMB 42557 are linked to QTL-1 or QTL-2, which QTLs confer ToLCNDV resistance in C. melo.**

| SNP markers | Nucleotide sequence | Indicative of |
|---|---|---|
| Cm_0205 (SEQ ID NO: 1) | TTGCAAGGTGGGCTGAGATGATGAGGAAGAGG GTTAAGAGAAGAAATCCTACAAACTTTA[C/T]TA ATGGTGACCATAGCCCTTAGAATCTCATTTAAT TAAACACCATTTTTCACTTAATCCA | QTL-1 |
| MEL_SNP_2208 (SEQ ID NO: 2) | GTGCATTTCATGAAAAAAATAGAAACTAAAAAG TGTGAATTTTAGAAAGT[T/C]CGACTTCAAATTG AAATAAAAAACTTAAAATTTAAAAAATAAAATTG TAA | QTL-1 |
| MEL_SNP_2224 (SEQ ID NO: 3) | TAAACCAACTATAACTTAGGGTTGTATAAATTTT CAAATGTCTGTTTCAA[G/T]GGTTACTTGCATAA ATGACAAAAAAAAAAAAAAACGTAAAATAAAAG TAT | QTL-1 |
| MEL_SNP_4105 (SEQ ID NO: 4) | AAATTTTCTCTCTAATACCATCTTAATTTTTCAAT TTTCTTTCTGTTACA[C/T]ATATGAATTTTACAAG GTATCAAATTTCACCTCAACCAAAAAAATGATTT | QTL-2 |
| MEL_SNP_4114 (SEQ ID NO: 5) | GAGAGTAATTAACACTTAGGTATAATTACGACG AATAAACTACAAAAGTA[G/T]TTTGAATTTGTGTT TTTTTGAGACACAAAGGTTTTTTAAAAATTTAT AT | QTL-2 |
| SFMe066 (SEQ ID NO: 6) | GCCTATCAGGAGTGAACTCAATCTCAATGGACA TGAAAGAGAAGAAGCTGACAGTAACAGGAGAC GTAGATCCAGTAGTGATTGTAAGCAAATTAAGG AA[A/G]ATTTGTCATACAACAATAGTTTCCGTTG GACCAGAGAAAGAAGAGAAGAAACCAGAACCA AAGAAAGATGAACCAAAGAAGGAAGATCCAAA AAA | QTL-2 |

**Table 2. Molecular SNP markers that in deposit number NCIMB 42557 are linked to QTL-1 or QTL-2, which QTLs confer ToLCNDV resistance in C. melo.**

| **SNP markers** | **Primer A1** | **Primer A2** | **Primer C1** |
|---|---|---|---|
| Cm_0205 (SEQ ID NO: 1) | SEQ ID NO: 7 | SEQ ID NO: 8 | SEQ ID NO: 9 |
| | GAAGGTGACCAA GTTCATGCTGTT AAGAGAAGAAAT CCTACAAACTTTA C | GAAGGTCGGAGTC AACGGATTGGTTAA GAGAAGAAATCCTA CAAACTTTAT | GAGATTCTAA GGGCTATGGT CACCAT |
| MEL_SNP_2208 (SEQ ID NO: 2) | SEQ ID NO: 10 | SEQ ID NO: 11 | SEQ ID NO: 12 |
| | GAAGGTGACCAA GTTCATGCTAATT TTAAGTTTTTTAT TTCAATTTGAAGT CGA | GAAGGTCGGAGTC AACGGATTAATTTTA AGTTTTTTATTTCAA TTTGAAGTCGG | AGAAACTAAA AAGTGTGAAT TTTAGAAAGT |
| MEL_SNP_2224 (SEQ ID NO: 3) | SEQ ID NO: 13 | SEQ ID NO: 14 | SEQ ID NO: 15 |
| | GAAGGTGACCAA GTTCATGCTTTTT TTTTTTTTGTCAT TTATGCAAGTAA CCC | GAAGGTCGGAGTC AACGGATTTTTTTT TTTTGTCATTTATGC AAGTAACCA | AGGGTTGTAT AAATTTTCAAA TGTCTGTTT |
| MEL_SNP_4105 (SEQ ID NO: 4) | SEQ ID NO: 16 | SEQ ID NO: 17 | SEQ ID NO: 18 |
| | GAAGGTGACCAA GTTCATGCTCAT CTTAATTTTTCAA TTTTCTTTCTGTT ACAC | GAAGGTCGGAGTC AACGGATTCATCTT AATTTTTCAATTTTC TTTCTGTTACAT | TGGTTGAGGT GAAATTTGATA CCTTGTAAA |
| MEL_SNP_4114 (SEQ ID NO: 5) | SEQ ID NO: 19 | SEQ ID NO: 20 | SEQ ID NO: 21 |
| | GAAGGTGACCAA GTTCATGCTGTA TAATTACGACGA ATAAACTACAAAA GTAG | GAAGGTCGGAGTC AACGGATTGTATAA TTACGACGAATAAA CTACAAAAGTAT | CCTTTGTGTCT CAAAAAAACA CAAATTCAA |
| SFMe066 (SEQ ID NO: 6) | SEQ ID NO: 22 | SEQ ID NO: 23 | SEQ ID NO: 24 |
| | GAAGGTGACCAA GTTCATGCTCAG TAGTGATTGTAA GCAAATTAAGGA AA | GAAGGTCGGAGTC AACGGATTCAGTAG TGATTGTAAGCAAA TTAAGGAAG | GTCCAACGGA AACTATTGTTG TATGACAA |

## Claims

1. A tomato leaf curl New Delhi virus (ToLCNDV) resistant plant of the species *Cucumis melo,* said plant comprising an introgression from a plant of the species *Cucumis melo* which seeds have been deposited with the NCIMB under accession number NCIMB 42557, wherein the introgression comprises a ToLCNDV-resistance-conferring QTL-1 located on linkage group (LG) 5 between physical positions 24,008,941 bp and 24,895,385 bp, or a ToLCNDV-resistant conferring part thereof and wherein said plant is not NCIMB 42557.

2. A ToLCNDV resistant plant according to claim 1 wherein the ToLCNDV-resistance-conferring QTL-1 is detectable by a molecular marker assay which detects at least the Single Nucleotide Polymorphism (SNP) marker MEL_SNP_2208 comprising the SEQ ID NO: 2.

3. A ToLCNDV resistant plant according to claim 2 wherein the ToLCNDV-resistance-conferring QTL-1 is further detectable by the SNP markers CM_0205 comprising the SEQ ID NO: 1, MEL_SNP_2224 comprising the SEQ ID NO: 3, or any combinations thereof.

4. A ToLCNDV resistant plant according to any one of claims 1-3 wherein QTL-1 or a part thereof is present in homozygous form.

5. A ToLCNDV resistant plant according to any one of claims 1-4 further comprising a ToLCNDV-resistance-conferring QTL-2 located on linkage group (LG) 9 between physical positions 22,847,688 bp and 23,556,554 bp, or a ToLCNDV-resistant conferring part thereof.

6. A ToLCNDV resistant plant according to claim 5 wherein the ToLCNDV-resistance-conferring QTL-2 is detectable by a molecular marker assay which detects at least the SNP marker MEL_SNP_4114 comprising the SEQ ID NO: 5.

7. A ToLCNDV resistant plant according to claim 6 wherein the ToLCNDV-resistance-conferring QTL-2 is further detectable by the SNP markers MEL_SNP_4105 comprising the SEQ ID NO: 4, SFMe066 comprising the SEQ ID NO: 6, or any combinations thereof.

8. A ToLCNDV resistant plant according to any of claims 1 to 7 wherein said plant of the species *Cucumis melo* is a melon cultivar, wild accession, and/or a plant of the group consisting of *Cucumis melo var. cantalupensis, Cucumis melo var. reticulates, Cucumis melo var. inodorus, Cucumis melo var. acidulus; Cucumis melo var. aegyptiacus; Cucumis melo var. ameri; Cucumis melo var. duripulposus; Cucumis melo var. hibernus; Cucumis melo var. makuwa* and *Cucumis melo var. microspermus.*

9. A part of a plant according to any one of the claims 1-8.

10. Plant part according to claim 9 wherein said part is a seed or fruit.

11. A ToLCNDV-resistance-conferring QTL-1 located on linkage group (LG) 5 between physical positions 24,008,941 bp and 24,895,385 bp, of NCIMB 42557, and wherein the ToLCNDV-resistance-conferring QTL-1 is detectable by a molecular marker assay which detects at least one of the SNP markers selecting from the list consisting of: CM_0205 comprising the SEQ ID NO: 1, MEL_SNP_2208 comprising the SEQ ID NO: 2, MEL_SNP_2224 comprising the SEQ ID NO: 3, or any combinations thereof.

12. Molecular marker, preferably a molecular SNP marker, present in *Cucumis melo* genome, which molecular marker is genetically linked to QTL that confers resistance to ToLCNDV in *Cucumis melo,* and wherein the molecular marker is **characterized by** any of the SEQ ID NO: 1-6 and wherein the QTL is preferably the QTL-1 and the QTL-2.

13. A method for identifying a ToLCNDV resistant *C. melo* plant comprising an introgression fragment located on linkage group (LG) 5, comprising:
a) providing a population of cultivated *C. melo* plants,
b) screening said population using a molecular marker assay which detects at least one SNP marker selected from the group consisting of: SNP marker MEL_SNP_2208 comprising the SEQ ID NO: 2; CM_0205 comprising the SEQ ID NO: 1, MEL_SNP_2208 comprising the SEQ ID NO: 2, MEL_SNP_2224 comprising the SEQ ID NO: 3, or any combinations thereof; and
c) identifying and/or selecting a plant comprising at least one of the SNP markers of step b).

14. Method according to claim 13 wherein the ToLCNDV resistant plant further comprises an introgression fragment located on linkage group (LG) 9 which is detected by screening the population using a molecular marker assay which detect at least one SNP marker selected from the group consisting of: MEL_SNP_4114 comprising the SEQ ID NO: 5, MEL_SNP_4105 comprising the SEQ ID NO: 4, SFMe066 comprising the SEQ ID NO: 6, or any combinations thereof.
